(19) Europäisches Patentamt
European Patent Office
Office européen des brevets

(11) **EP 3 240 436 B1**

(12) **FASCICULE DE BREVET EUROPEEN**

(45) Date de publication et mention
de la délivrance du brevet:
**13.05.2020 Bulletin 2020/20**

(21) Numéro de dépôt: **15830827.0**

(22) Date de dépôt: **30.12.2015**

(51) Int Cl.:
***A23L 33/17*** ***(2016.01)***

(86) Numéro de dépôt international:
**PCT/FR2015/053784**

(87) Numéro de publication internationale:
**WO 2016/108036 (07.07.2016 Gazette 2016/27)**

(54) **COMPOSITION COMPORTANT DE LA CHITINE ET DES PROTÉINES DIGESTIBLES**

ZUSAMMENSETZUNG MIT CHITIN UND VERDAULICHEN PROTEINEN

COMPOSITION CONTAINING CHITIN AND DIGESTIBLE PROTEINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **31.12.2014 FR 1463512**
**20.10.2015 FR 1560013**

(43) Date de publication de la demande:
**08.11.2017 Bulletin 2017/45**

(73) Titulaire: **Ynsect**
**91058 Évry-Courcouronnes Cedex (FR)**

(72) Inventeurs:
• **ARMENJON, Benjamin**
**75013 Paris (FR)**
• **BEREZINA, Nathalie**
**75005 Paris (FR)**
• **LAURENT, Sophie**
**44200 Nantes (FR)**
• **HUBERT, Antoine**
**94140 Alfortville (FR)**
• **SOCOLSKY, Cecilia**
**59800 Lille (FR)**
• **SANCHEZ, Lorena**
**91260 Juvisy-sur-Orge (FR)**

(74) Mandataire: **Santarelli**
**49, avenue des Champs-Elysées**
**75008 Paris (FR)**

(56) Documents cités:
**CN-A- 1 297 691        CN-A- 101 116 471**
**CN-A- 101 292 737      CN-B- 101 144 097**

• **Anonymous: "Insects for food and feed", Food and Agricultural Organization of the United Nations , 14 janvier 2014 (2014-01-14), page 1, XP002757258, Extrait de l'Internet: URL:http://www.fao.org/edible-insects/8462 5/en/ [extrait le 2016-05-03]**
• **Hervé Guénot: "Des insectes dans la farine", JDD Paris , 19 décembre 2012 (2012-12-19), pages 1-2, XP002757230, Extrait de l'Internet: URL:http://www.lejdd.fr/JDD-Paris/Actualit e/Des-insectes-dans-la-farine-581928 [extrait le 2016-05-02]**

**Description**

La présente invention se rapporte à une composition comportant des protéines et de la chitine. Elle vise également un procédé de préparation de cette composition et son utilisation dans l'alimentation humaine ou animale, et plus particulière dans l'alimentation des poissons.

L'aquaculture est aujourd'hui l'un des secteurs les plus dynamiques de l'industrie alimentaire. La forte demande en poissons a eu pour conséquence d'augmenter significativement le prix des aliments destinés à l'élevage des poissons.

L'un des produits les plus utilisé dans l'alimentation des poissons est la farine de poissons. En effet, la farine de poisson est une des principales sources de protéines dans les aliments aquacoles. C'est une farine très riche en protéines animales (riche en acides aminés type lysine et méthionine) faciles à digérer. Une demande croissante accompagnée d'une offre limitée a eu pour conséquence d'en augmenter significativement son prix, engendrant un risque pour la croissance durable de l'aquaculture. Ainsi, il y a une forte demande pour des sources alternatives de protéines de qualité élevée et, dans la mesure du possible, renouvelables, pour les aliments aquacoles.

Les farines d'insectes proposent des sources protéiques naturelles de remplacement et la possibilité d'être produit en masse avec une empreinte écologique minimale. En particulier, certains insectes tels que *Tenebrio molitor*, présentent l'intérêt de pouvoir être adaptés à une production en masse intensive.

L'article extrait d'internet « Insects for food and feed » par la Food and Agriculture Organization of the United Nations (http://www.fao.org/edible-insects/84625/en/) divulgue des informations générales sur la valeur nutritionnelle des insectes.

L'article extrait d'internet « Des insectes dans la farine » par Hervé Guénot (http://www.lejdd.fr/JDD-Paris/Des-insectes-dans-la-farine-581928) divulgue l'existence de farines provenant de différentes biomasses telles que des farines d'insectes.

L'article de Sandra G.F. Bukkens (1997), « The nutritional value of edible insects », Ecology of Food and Nutrition, 36:2-4, 287-319 divulgue les valeurs nutritionnelles de différents insectes, notamment leurs teneurs en protéines brutes, matière grasse, cendres et fibres brutes.

La demande US2008/0075818 décrit un procédé de préparation d'une farine d'insectes.

Le document « Edible insects. Future prospects for food and feed security », FAO Forestry paper 171, par la Food and Agriculture Organization of the United Nations publié en 2013 décrit différents aspects des insectes consommables.

Toutefois, les résultats d'essais de substitution de la farine poissons par diverses farines d'insectes s'avèrent mitigés. Dans le cas où la substitution s'avère possible, celle-ci n'excède généralement pas les 50%, au-delà de cette teneur des effets néfastes sur la croissance des poissons étant observés.

Le travail des inventeurs a permis de mettre en évidence qu'une composition spécifique pouvait être avantageusement utilisée en remplacement d'une farine de poissons dans l'alimentation aquacole.

La présente invention concerne donc une composition comportant au moins 67% en poids de protéines brutes, au moins 5% en poids de chitine, les pourcentages en poids étant donnés sur le poids total de composition, et 85% en poids de protéines digestibles sur le poids total de protéines brutes.

On notera que dans le cadre de la présente demande, et sauf stipulation contraire, les gammes de valeurs indiquées s'entendent bornes incluses.

La quantification des « protéines brutes » est bien connue de l'homme du métier. A titre d'exemple, on peut citer la méthode Dumas ou la méthode Kjeldhal. De préférence, la méthode Dumas, correspondant à la norme NF EN ISO 16634-1 (2008) est utilisée.

Dans toute la demande, lorsqu'aucune date n'est précisée pour un règlement, une norme ou une directive, il s'agit du règlement, de la norme ou de la directive en vigueur à la date de dépôt.

Préférentiellement, la composition comporte 68% en poids de protéines brutes, plus préférentiellement 70% en poids de protéines brutes, les pourcentages en poids étant donnés sur le poids total de composition.

Par « protéines digestibles », on vise les protéines digestibles déterminées par la digestibilité pepsique. La quantification des protéines digestibles s'effectuera de préférence par la méthode décrite dans la directive 72/199/CE.

Préférentiellement, la composition comporte 86%, plus préférentiellement 88% en poids de protéines digestibles sur le poids total de protéines brutes.

Selon l'invention, par « chitine », on entend tout type de dérivé chitinique, c'est-à-dire de dérivé de polysaccharides comportant des unités N-acétyl-glucosamine et des unités D-glucosamines, en particulier les copolymères chitine-polypeptides (parfois désignés sous l'appellation « composite chitine-polypeptides »). Ces copolymères peuvent également être associés à des pigments, souvent de type mélanine.

La chitine serait le deuxième polymère le plus synthétisé dans le monde vivant après la cellulose. En effet, la chitine est synthétisée par de nombreuses espèces du monde vivant: elle constitue en partie l'exosquelette des crustacés et des insectes et la paroi latérale qui entoure et protège les champignons. Plus particulièrement, chez les insectes, la chitine constitue ainsi 3 à 60% de leur exosquelette.

La détermination du taux de chitine est effectuée par extraction de celle-ci. Une telle méthode peut être la méthode ADAC 991.43 décrite à l'exemple 2, et est une méthode préférée pour cette détermination.

Préférentiellement, la composition comporte entre 5 et 16% en poids de chitine, plus préférentiellement entre 8 et 14% de chitine, les pourcentages en poids étant donnés sur le poids total de composition.

Les compositions de l'art antérieur susceptibles de contenir à la fois des protéines et de la chitine sont en général des compositions obtenues à partir d'insectes et/ou de crustacés. Toutefois, les forts taux de protéines brutes et de protéines digestibles de la composition selon l'invention ne peuvent être obtenus que par un procédé de traitement des insectes et/ou des crustacés, comportant une étape d'hydrolyse. Or, une étape d'hydrolyse a pour effet d'abaisser le taux de chitine à une teneur de l'ordre de 5% en poids, telle qu'inférieure à 5% en poids, sur le poids total de la composition.

Or, la chitine est souvent considérée comme une sorte de facteur anti-nutritionnel car difficile à digérer. Ceci explique que pour des applications dans le domaine agro-alimentaires, des compositions à base d'insectes sont dé-chitinées, c'est-à-dire qu'une étape de retrait de la chitine est effectuée. Or, le travail des inventeurs a également permis de démontrer que, contrairement aux idées reçues, la chitine n'avait pas d'impact sur la croissance de poissons nourris avec une composition selon l'invention, comportant un taux non négligeable de chitine (voir Exemple 4 ci-après). Au contraire, la composition selon l'invention peut avantageusement remplacer non seulement partiellement mais également dans sa totalité, une farine de poissons dans un aliment aquacole. En effet, la composition selon l'invention permet d'améliorer la croissance d'animaux nourris avec cette composition.

En outre, lors du processus de fabrication des aliments, l'introduction de la composition selon l'invention présente également certains avantages : réduction en pertes en vitamines hydrosolubles lors d'éventuels traitement thermiques et réduction de l'énergie nécessaire lors d'une éventuelle étape d'extrusion.

De préférence, la composition selon l'invention présente un taux d'humidité résiduel compris entre 2 et 15%, de préférence entre 5 et 10%, plus préférentiellement, entre 6 et 8%. Ce taux d'humidité peut par exemple être déterminé selon la méthode issue du règlement CE 152/2009 du 27-01-2009 (103 °C / 4 h).

Avantageusement, la composition selon l'invention présente une teneur en cendres inférieure ou égale à 4% en poids sur le poids total de composition, et encore plus avantageusement, inférieure ou égale à 3,5%.

Les cendres constituent le résidu résultant de la combustion de la composition selon l'invention.

La méthode de détermination de la teneur en cendres est bien connue de l'homme du métier. De préférence, les cendres ont été déterminées selon la méthode relevant du règlement CE 152/2009 du 27-01-2009.

La teneur en matière grasse de la composition selon l'invention est de préférence comprise entre 5 et 20% en poids sur le poids total de composition, plus préférentiellement entre 9 et 17%.

Les méthodes de détermination de la teneur en matière grasse sont bien connues de l'homme du métier. A titre d'exemple et de manière préférée, la détermination de cette teneur sera effectuée en suivant la méthode du règlement CE 152/2009.

Comme indiquée ci-avant, la composition selon l'invention peut être obtenue à partir d'insectes.

Par « obtenue » à partir d'insectes, on entend plus particulièrement une composition obtenue uniquement à partir d'insectes et éventuellement d'eau. La composition résulte d'un traitement mécanique, thermique, à l'exclusion de tout traitement chimique (autre que par l'eau) des insectes.

Plus particulièrement, la composition est une farine d'insectes. Par farine d'insectes, on vise une poudre ayant une taille de particules acceptable pour l'alimentation humaine ou animale. Par « taille de particules acceptable pour l'alimentation humaine ou animale », on vise une taille de particules comprise entre 100 $\mu$m et 1,5 mm, préférentiellement comprise entre 300 $\mu$m et 1 mm, plus préférentiellement entre 500 et 800$\mu$m.

Les insectes préférés pour la préparation d'une telle farine sont par exemple les coléoptères, les diptères, les lépidoptères, les isoptères, les orthoptères, les hyménoptères, les blattoptères, les hémyptères, les hétéroptères, les éphéméroptères et les mécoptères, de préférence, les coléoptères, les diptères, les orthoptères, les lépidoptères ou leurs mélanges.

Préférentiellement, les insectes sont choisis parmi le groupe constitué par *Tenebrio molitor*, *Hermetia illucens*, *Galleria mellonella*, *Alphitobius diaperinus*, *Zophobas morio*, *Blattera fusca*, *Tribolium castaneum*, *Rhynchophorus ferrugineus*, *Musca domestica*, *Chrysomya megacephala*, *Locusta migratoria*, *Schistocerca gregaria*, *Acheta domesticus*, *Samia ricini* ou leurs mélanges, et plus préférentiellement encore, *Tenebrio molitor*.

Avantageusement, la composition selon l'invention comporte entre 35 et 65% en poids de protéines solubles par rapport au poids total de protéines brutes, et au moins 50% des protéines solubles ont une taille inférieure ou égale à 12400g/mol.

Par « protéines solubles », on entend, parmi les protéines brutes, celles qui sont solubles dans une solution aqueuse dont le pH est compris entre 6 et 8, avantageusement entre 7,2 et 7,6.

De préférence, la solution aqueuse est une solution tampon dont le pH est compris entre 6 et 8, avantageusement entre 7,2 et 7,6. Préférentiellement, la solution tampon est une solution tampon phosphate NaCl, dont le pH est égal 7,4 +/- 0,2.

La digestibilité des protéines chez l'homme et les animaux est fortement conditionnée par la taille des protéines. En nutrition animale, il est courant de réduire la taille des protéines, afin de faciliter la digestion des animaux. Cette réduction de la taille des protéines se fait généralement par des procédés d'hydrolyse (par exemple enzymatique), dont la mise en œuvre est particulièrement coûteuse.

La composition selon l'invention, obtenue par un procédé ne faisant pas intervenir d'hydrolyse, comporte une quantité importante de protéines solubles dont la taille est suffisamment réduite pour faciliter la digestion des animaux. La composition selon l'invention présente en outre l'avantage de pouvoir être préparée à moindre coût.

Avantageusement, la composition selon l'invention comporte entre 30 et 60% en poids, de préférence entre 35 et 55% en poids de protéines solubles par rapport au poids total de protéines brutes.

De préférence, au moins 60%, préférentiellement au moins 70% des protéines solubles ont une taille inférieure ou égale à 12400 g/mol.

Plus particulièrement, les protéines solubles ont une taille comprise entre 6500 et 12400 g/mol.

Avantageusement, moins de 10%, de préférence moins de 8%, plus préférentiellement moins de 5%, et encore plus préférentiellement 0% des protéines solubles ont une taille supérieure ou égale à 66000 g/mol.

Une telle répartition des protéines solubles est mise en évidence dans l' Exemple 6.

L'invention divulgue également un procédé de préparation d'une composition selon l'invention.

Le procédé de préparation d'une composition selon l'invention comporte une étape de pressage des insectes.

L'objectif du pressage est de déshuiler les insectes et donc d'obtenir un gâteau de presse ayant une teneur en huile (ou matière grasse) inférieure ou égale à 20% en poids sur le poids sec du gâteau de presse, préférentiellement, inférieure ou égale à 17%.

L'étape de pressage est plus amplement décrite dans l'étape 2 du procédé de préparation détaillé ci-après.

En particulier, il est possible d'effectuer un pressage à chaud ou à froid. De préférence, une presse mono-vis est utilisée.

Plus particulièrement, le procédé de préparation selon l'invention comporte les étapes suivantes :

i) abattage d'insectes
ii) pressage des insectes pour obtenir un gâteau de presse,
iii) séchage du gâteau de presse, et
iv) broyage du gâteau de presse.

L'abattage d'insectes peut être effectué par ébouillantage ou blanchiment, comme cela est plus amplement décrit ci-après dans l'étape 1 du procédé détaillé.

De même, le broyage est plus amplement décrit dans l'étape 4 du procédé détaillé.

Enfin, le procédé de préparation selon l'invention comporte en outre une étape de séchage du gâteau de presse.

L'étape de séchage est réalisée après l'étape de pressage et avant l'étape de broyage.

Le séchage est plus amplement décrit dans l'étape 3 du procédé détaillé.

**Procédé détaillé de préparation d'une composition selon l'invention**

**• Etape** 1 : **abattage des insectes**

Cette étape 1 d'abattage peut avantageusement s'effectuer par ébouillantage ou par blanchiment. Cette étape 1 permet d'abattre les insectes tout en abaissant la charge microbienne (réduction du risque d'altération et sanitaire) et en inactivant les enzymes internes des insectes pouvant déclencher une autolyse, et ainsi un brunissement rapide de ceux-ci.

Pour l'ébouillantage, les insectes, de préférence des larves, sont ainsi ébouillantés à l'eau pendant 2 à 20 min, préférentiellement, 5 à 15 min. De préférence, l'eau est à température comprise entre 95 à 105°C, préférentiellement 100°C.

La quantité d'eau introduite lors de l'ébouillantage est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Pour le blanchiment, les insectes, de préférence des larves, sont blanchis à la vapeur (buses ou lit de vapeur) à une température comprise entre 80 et 130°C, de préférence entre 90 et 120°C, plus préférentiellement entre 95 et 105°C, préférentiellement 98°C ou bien à l'eau à une température comprise entre 95 et 105°C, préférentiellement 100°C (par buses d'aspersion) ou en mode mixte (eau + vapeur) à une température comprise entre 80 et 130°C, de préférence entre 90 et 120°C, plus préférentiellement entre 95 et 105°C, préférentiellement 98°C. Le temps de séjour dans la chambre de blanchiment est compris entre 1 à 15 minutes, préférentiellement entre 3 et 7 min.

**• Etape (optionnelle) : broyage**

Les insectes sont retirés de la cuve d'ébouillantage ou de la chambre de blanchiment, ils sont ensuite tamisés (ou égouttés), et placés dans un broyeur, tel qu'un broyeur mixeur à couteaux, permettant de réduire les insectes en particules.

Afin de faciliter le broyage, une quantité d'eau peut être ajoutée. Cette quantité d'eau est similaire à celle introduite lors de l'étape 1 d'ébouillantage : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1. Il est également possible de garder l'eau d'ébouillantage et/ou l'eau résultant du blanchiment pour effectuer cette étape.

De préférence, à l'issue du broyage, la taille des particules d'insectes est inférieure à 1 cm (plus grande taille de particule observable à l'aide d'un microscope), de préférence inférieure à 0,5 cm. Préférentiellement, la taille des particules est comprise entre 300 $\mu$m et 3 mm, plus préférentiellement entre 500 $\mu$m et 1 mm. Il n'est pas nécessaire de réduire excessivement la taille des particules, par exemple à une taille inférieure à 250 $\mu$m.

**• Etape 2 : pressage**

Les insectes issus de l'étape 1 d'abattage ou la pâte humide issue de l'étape optionnelle de broyage est ensuite placée dans une presse selon un mode opératoire qui permet de presser et séparer un jus comportant à la fois une fraction huileuse et une fraction protéique.

De préférence, l'étape de pressage permet d'obtenir un gâteau de presse comportant une teneur en huile inférieure ou égale à 20% en poids sur le poids sec du gâteau de presse, préférentiellement, inférieure ou égale à 17%, plus préférentiellement encore inférieure ou égale à 15%.

De même, l'étape de pressage permet d'obtenir un gâteau de presse présentant une teneur en matière sèche comprise entre 30% et 60%, préférentiellement, comprise entre 40% et 55%, et plus préférentiellement comprise entre 45% et 55%.

Tout système de presse peut être utilisé pour réaliser l'étape de pressage, tel que par exemple, une presse mono-vis ou bi-vis (presse bi-vis de type Angel), un filtre-presse (filtre-presse de type Choquenet), une presse à plateaux, etc. Ces systèmes sont bien connus de l'homme du métier qui est à même de déterminer les conditions de pressage afin d'obtenir les teneurs en huile et/ou en eau mentionnées ci-avant.

En particulier, il est possible d'effectuer un pressage à chaud ou à froid. Avantageusement, le pressage sera effectué à chaud, ce qui permet d'augmenter le déshuilage du gâteau de presse. En particulier, un pressage à chaud permet l'obtention d'un gâteau de presse comportant une teneur en huile inférieure ou égale à 17% en poids sur le poids sec du gâteau de presse, de préférence, inférieure ou égale à 15%.

**• Etape 3 : séchage**

Le gâteau de presse est ensuite séché par les technologies classiques connues par l'homme de métier. Le séchage peut être direct ou indirect (sécheur en couche mince, « paddle dryer », « tubular dryer », « disc-dryer », etc.) à une température comprise entre 60°C et 200°C, pendant une durée de 15 min à 24 heures. A titre d'exemple, le gâteau de presse peut être disposé et séché à l'air ventilé/brassé à une température comprise entre 80 et 100°C, préférentiellement à 90°C pendant une durée comprise entre 3 et 7 heures, préférentiellement 5 heures.

L'objectif de cette étape de séchage est l'obtention d'un gâteau de presse ayant un taux d'humidité compris entre 2 et 15%, de préférence entre 5 et 10%, plus préférentiellement encore entre 4 et 8%.

**• Etape 4 : broyage final**

Le gâteau de presse séché est ensuite placé dans un broyeur, tel qu'un broyeur à marteaux, permettant de réduire le gâteau de presse en particules.

Avantageusement, à l'issue de ce broyage final, la taille des particules d'insectes est inférieure à 0,5 cm (plus grande taille de particule observable à l'aide d'un microscope), de préférence de l'ordre de 1 mm. Plus particulièrement, la taille de particules est comprise entre 300 $\mu$m et 1 mm, encore plus préférentiellement entre 500 et 800 $\mu$m.

La succession de ces quatre étapes permet d'obtenir une composition selon l'invention, comportant un fort taux de protéines brutes et de protéines digestibles tout en maintenant un taux de chitine de l'ordre d'au moins 5% en poids sur le poids total de la composition.

Comme indiqué ci-avant, l'étape de pressage peut être réalisée à froid ou à chaud.

A titre d'exemple de procédé d'obtention d'une composition selon l'invention, impliquant un pressage à froid : Des larves, par exemple de *T. molitor,* sont introduites dans un bêcher contenant 200 mL d'eau préalablement portée à ébullition, et abattues par ébouillantage au bain-marie à 100 °C. Après 5 minutes, le bécher est retiré du bain-marie, les larves sont essorées, puis mixées avec un volume d'eau de 200 mL. Le liquide ainsi obtenu est passé dans une presse de type bi-vis. Le gâteau de presse ainsi obtenu est séché 24 heures dans une étuve à 70 °C, puis broyé à 250 $\mu$m.

A titre d'exemple de procédé d'obtention d'une composition selon l'invention, impliquant un pressage à chaud : Des larves, par exemple de *T. molitor,* sont introduites dans une chambre de blanchiment et blanchies à la vapeur pendant 5 min à 100°C. Les larves ainsi blanchies sont ensuite introduites dans une presse de type "assèchement" adaptée aux produits chargés en eau. Le gâteau de presse ainsi obtenu est séché 5 heures dans une étuve à 90 °C, puis broyé dans un broyeur à marteaux à 1 mm.

Selon un premier mode de réalisation du procédé selon l'invention, l'étape de pressage est précédée d'une étape de broyage des insectes.

L'invention concerne donc un procédé de préparation d'une composition selon l'invention comportant les étapes suivantes :

i) abattage des insectes,
ii) pressage des insectes pour obtenir un gâteau de presse,
iii) séchage du gâteau de presse, et
iv) broyage du gâteau de presse,

dans lequel l'étape de pressage est précédée d'une étape de broyage des insectes.

Un avantage de l'étape de broyage des insectes préalable au pressage est plus amplement décrit dans l'Exemple 5.

Selon un second mode de réalisation du procédé selon l'invention, l'étape de pressage des insectes est réalisée à chaud.

L'invention concerne donc un procédé de préparation d'une composition selon l'invention comportant les étapes suivantes :

i) abattage des insectes,
ii) pressage des insectes pour obtenir un gâteau de presse,
iii) séchage du gâteau de presse, et
iv) broyage du gâteau de presse,

dans lequel l'étape de pressage est réalisée à chaud.

Comme indiqué ci-avant, le pressage à chaud permet l'obtention d'un gâteau de presse comportant une teneur en huile inférieure ou égale à 17% en poids sur le poids sec de gâteau de presse, de préférence inférieure ou égale à 15%.

Selon un troisième mode de réalisation du procédé selon l'invention, l'étape de broyage du gâteau de presse est réalisée à une taille de particules comprise entre 300 $\mu$m et 1 mm, de préférence entre 500 et 800 $\mu$m.

L'invention concerne donc un procédé de préparation d'une composition selon l'invention comportant les étapes suivantes :

i) abattage des insectes,
ii) pressage des insectes pour obtenir un gâteau de presse,
iii) séchage du gâteau de presse, et
iv) broyage du gâteau de presse,

dans lequel l'étape de broyage du gâteau de presse est réalisé à une taille de particules comprise entre 300 $\mu$m et 1 mm.

Plus particulièrement, dans ce troisième mode de réalisation du procédé selon l'invention, l'étape de pressage des insectes peut être réalisée à chaud. Alternativement, l'étape de pressage pourra être précédée d'une étape de broyage des insectes.

L'invention concerne enfin l'utilisation d'une composition selon l'invention dans l'alimentation humaine ou animale.

Avantageusement, la composition selon l'invention peut être utilisée dans l'alimentation des animaux de compagnie tels que les chiens, les chats, les oiseaux, les poissons, les reptiles, les rongeurs.

Plus particulièrement, la composition selon l'invention peut être utilisée dans l'aquaculture (poissons, crustacés, mollusques, coquillages), l'alimentation des volailles (poulet, dinde, gibier tel que caille, faisan, outarde), porcins, ruminants (bovins, ovins, caprins, équins), visons.

Enfin, la composition selon l'invention peut être avantageusement utilisée en remplacement d'une farine protéique.

Par farine protéique, on vise plus particulièrement une farine de poisson, un poudre de lait ou de lactosérum, une farine de concentré de soja (« CSP »), de la farine de viande, telle que par exemple de type farine de volailles (« Poultry Meal »).

Le remplacement peut être partiel ou total.

Préférentiellement, la composition selon l'invention est utilisée en remplacement partiel ou total d'une farine de poisson, tel qu'un remplacement à 50 ou 100%.

D'autres caractéristiques et avantages de l'invention, apparaîtront dans les exemples qui suivent, donnés à titre illustratif, avec référence à :

- La Figure 1, qui est un diagramme illustrant les variations de températures de l'eau et des niveaux d'oxygène dissout dans les réservoirs où ont été élevés les truites nourries avec différentes dose de composition selon l'invention,
- La Figure 2, qui comporte deux diagrammes illustrant l'impact sur le poids corporel final (Fig. 2A) et l'indice de consommation (Fig. 2B) des truites nourries avec différentes dose de composition selon l'invention.
- La Figure 3, qui illustre la répartition des lipides issus de l'insecte retrouvée dans le jus et le gâteau de presse obtenus par un procédé comportant une étape de pressage ou une étape de broyage puis de pressage, et
- La Figure 4, qui est un diagramme représentant l'analyse par chromatographie d'exclusion stérique de la taille des protéines de la composition selon l'invention.

EXEMPLE 1 : **Procédé de préparation d'une composition selon l'invention**

La composition selon l'invention est préparée à partir de larves de *Tenebrio molitor.* A réception des larves, ces dernières peuvent être stockées à 4°C pendant 0 à 15 jours dans leurs bacs d'élevages avant l'abattage sans dégradation majeure. Le poids des larves (âge) des larves utilisées est variable et par conséquent leur composition peut varier, comme cela est illustré dans le Tableau 1 ci-après :

**Tableau 1** : Composition biochimique des larves de *Tenebrio molitor* selon leur poids.

| Biomasse (Insectes) | mg | 23 | 35 | 58 | 80 | 108 | 154 |
|---|---|---|---|---|---|---|---|
| **Matière sèche** | %* | 34 | 34 | 34,2 | 37,9 | 39,6 | 39,5 |
| **Cendres** | %* | 1,59 | 1,52 | 1,6 | 1,75 | 1,67 | 1,43 |
| **Protéines brutes** | %* | 22,6 | 22,2 | 22 | 23,2 | 23,1 | 23,2 |
| **Lipides** | %* | 6,62 | 6,88 | 7,98 | 10,3 | 10,9 | 11,7 |
| * Les % sont exprimés en poids sec par rapport au poids humide de larves. | | | | | | | |

• **Etape 1 : Blanchiment des insectes**

Les larves vivantes (+4°C à + 25°C) sont convoyées en couche d'épaisseur comprise entre 2 et 10 cm, sur un tapis à bande perforé (1mm) jusqu'à une chambre de blanchiment. Les insectes sont ainsi blanchis à la vapeur (buses ou lit de vapeur) à 98°C ou bien à l'eau à 100°C (buses d'aspersion) ou en mode mixte (eau + vapeur). Le temps de séjour dans la chambre de blanchiment est compris entre 1 à 15 minutes, idéalement 5 min.

La température des larves en sortie de blanchiment est comprise entre 75°C et 98°C.

• **Etape 2 : Pressage**

Les larves, une fois blanchies, sont convoyées jusqu'à la trémie d'alimentation d'une presse mono-vis continue. Les larves lors du passage en presse sont maintenues à une température supérieure à 70°C pour augmenter les rendements de déshuilage. Le principe de déshuilage est de mettre en pression la matière à l'intérieur d'une cage cylindrique au moyen d'un arrangement de vis et de bagues disposées sur l'axe central. La cage est tapissée intérieur de barreaux répartis en section et maintenus écartée par des espaces d'épaisseurs différentes suivantes la zone de travail. Les interstices ainsi ménagés permettent l'écoulement d'une fraction huile et limitant le passage de la matière dite « sèche », la fraction protéique, que l'on appellera « gâteau de presse », participant de la sorte à la mise en pression.

Les rendements de pressage obtenus sont compris entre 48 et 55 %.

$$R_{gâteau}= (masse_{gâteau} / masse_{jus} + masse_{gâteau})$$

Le gâteau de presse obtenu contient 35 à 40 % de matières sèches, 67 à 75 % de protéines et 13 à 17 % de matières grasses, les pourcentages en poids étant donnés sur le poids sec de gâteau de presse.

• **Etape 3 : Séchage**

Le gâteau de presse est ensuite disposé sur plateau en couche fine (2 cm environ) et, est séché en air ventilé/brassé à 90°C pendant 5 heures afin d'obtenir un gâteau de presse ayant une teneur en matière sèche supérieure à 92%.

Cette étape permet de se prémunir de toute contamination ayant eu lieu depuis l'abattage.

L'activité en eau (Aw) en sortie séchage est de 0,35. Les résultats microbiologiques montrent une absence de *Salmonella spp* (méthode : IRIS Salmonella BKR 23/07-10/11) et des valeurs en Entérobactéries inférieures à 10 UFC/g (méthode : NF ISO 2128-2, décembre 2004, 30 °C et 37 °C).

• **Etape 4** : **Broyage**

Le gâteau de presse séché, comportant majoritairement des protéines, est enfin broyé à l'aide d'un broyeur à marteau continu (6 mobiles réversibles - épaisseur 8 mm). Le broyeur est alimenté par une trémie avec trappe de réglage de débit (180kg/h). La grille perforée utilisée pour contrôler la granulométrie en sortie est de 0,8 mm. La vitesse de rotation du moteur est de 3000 tr/min (motorisation électrique, puissance absorbée 4 kW (5,5 CV)).

**EXEMPLE 2** : Caractérisation **de** la **composition selon l'invention**

La composition préparée à l'Exemple 1 a été caractérisée.

**1. Analyses**

1.1 Détermination du taux d'humidité

Le taux d'humidité est déterminé selon la méthode issue du règlement CE 152/2009 du 27-01-2009 (103 °C / 4 h).

1.2 Détermination de la quantité de protéines brutes

Les protéines brutes sont déterminées selon la méthode, dite de Dumas, et correspondant à la norme NF EN ISO 16634-1 (2008).

1.3 Détermination de la quantité de chitine

Les fibres alimentaires de la farine des insectes sont essentiellement composées de chitine, cette dernière a donc été dosée suivant la méthode ADAC 991.43. Les valeurs ainsi obtenues sont par conséquent légèrement surestimées.

1.4 Détermination de la quantité de matière grasse

La matière grasse a été déterminée suivant la méthode du règlement CE 152/2009.

1.5 Détermination de la quantité de cendres

Les cendres brutes ont été déterminées selon la méthode relevant du règlement CE 152/2009 du 27-01-2009.

1.6 Détermination de la quantité de phosphore

Le phosphore est dosé par ICP (« induced coupled plasma ») avec étalonnage interne.

1.7 Détermination de l'énergie

La valeur énergétique est obtenue avec les coefficients du règlement UE 1169/201.

1.8 Détermination des quantités en acides aminés et en acides gras

Cette détermination a été effectuée par chromatographie en phase gazeuse après hydrolyse et dérivatisation des acides aminés et acides gras respectivement.

1.9 Détermination de la digestibilité pepsique

La digestibilité pepsique est mesurée par la méthode décrite dans la directive 72/199/CE.

2. Résultats

La composition selon l'invention est détaillée dans le Tableau 2 ci-après.

Tableau 2: composition

| Macronutriment | Unité | Composition |
|---|---|---|
| Humidité | %* | 5,32 |
| Protéine | %* | 67,09 |
| Chitine | %* | 8,0 |
| Matière grasse | %* | 13,6 |
| Cendre | %* | 3,21 |
| Phosphore total | %* | 0,75 |
| Énergie | MJ/kg | 23,74 |
| | | |
| Acides aminés | Unité | Composition |
| Arginine | %* | 2,56 |
| Histidine | %* | 1,39 |
| Isoleucine | %* | 2,11 |
| Leucine | %* | 3,99 |
| Lysine | %* | 3,32 |
| Thréonine | %* | 1,87 |
| Valine | %* | 2,91 |
| Méthionine | %* | 1,43 |
| Acides gras | Unité | Composition |
| C12:0 | %* | 0,03 |
| C14:0 | %* | 0,22 |
| C15:0 | %* | 0,01 |
| C16:0 | %* | 1,33 |
| C16:1 | %* | 0,05 |
| C16:1n-7 | %* | 0,16 |
| C17:0 | %* | 0,02 |
| C17:1 | %* | 0,01 |
| C18:0 | %* | 0,35 |
| C18:1n-9 | %* | 3,03 |
| C18:1n-7 | %* | 0,04 |

(suite)

| Acides gras | Unité | Composition |
|---|---|---|
| C18:2n-6 | %* | 2,96 |
| C18:2tn-6 | %* | 0,02 |
| C18:3n-3 | %* | 0,14 |
| C20:0 | %* | 0,02 |
| C20:1n-9 | %* | 0,01 |
| C20:2n-6 | %* | 0,01 |
| Cystéine | %* | 0,63 |
| Phénylalanine | %* | 1,98 |
| Tyrosine | %* | 2,68 |
| Taurine | %* | 0,42 |
| Acide aspartique + asparagine | %* | 4,51 |
| Acide glutamique + glutamine | %* | 6,36 |
| Alanine | %* | 3,83 |
| Glycine | %* | 2,54 |
| Proline | %* | 3,18 |
| Serine | %* | 2,94 |
| C22:0 | %* | 0,01 |
| * Les pourcentages en poids sont exprimés sur le poids total de composition. | | |

Par ailleurs, une digestibilité pepsique de 90+/-2% est obtenue.

**EXEMPLE 3** : **Procédé alternatif de préparation d'une composition selon l'invention**

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. Le gâteau de presse ainsi obtenu est séché 24 heures dans une étuve à 70 °C, puis broyé à 250 $\mu$m. On obtient ainsi une composition selon l'invention.

**EXEMPLE 4** : **Introduction de la composition selon l'invention dans l'alimentation de poisson**

Dans le présent exemple, l'effet de l'inclusion alimentaire d'une composition selon l'invention sur la croissance, l'apport alimentaire, la conversion alimentaire, la composition corporelle et la digestibilité apparente des nutriments chez la truite arc-en-ciel a été étudié.

**1. Matériel et méthodes**

**1.1. Composition selon l'invention**

La composition mise en œuvre dans cet exemple est celle obtenue selon l'Exemple 1 et plus amplement décrite dans l'Exemple 2.

**1.2. Régimes expérimentaux**

Un régime à base de farine de poisson (CTRL) a été formulé avec des ingrédients pratiques pour répondre aux besoins nutritionnels connus des truites arc-en-ciel juvéniles. Ce régime CTRL est composé de 25% de farine de poisson, 8% d'autres sources protéiques d'origine marine (farine de calmar et farine de krill), tandis que les sources de protéines restantes étaient un concentré de protéine de soja, de gluten de blé et de gluten de maïs. Sur la base de cette formulation, quatre régimes de tests (Y5, Y7,5, Y15 et Y25) ont été formulés, dans lesquels la farine de poisson a été remplacée par la composition selon l'invention à des taux respectifs de 20, 30, 60 et 100% (voir le Tableau 3 ci-dessous).

Tableau 3 : Formulation et composition des régimes expérimentaux.

| Ingrédients en %*: | CTRL | Y5 | Y7,5 | Y15 | Y25 |
|---|---|---|---|---|---|
| Farine de poisson LT70[1] | 25,00 | 20,00 | 17,50 | 10,00 | 0,00 |
| Farine de krill[2] | 3,00 | 3,00 | 3,00 | 3,00 | 3,00 |
| Farine de calmar[3] | 5,00 | 5,00 | 5,00 | 5,00 | 5,00 |
| Composition selon l'invention | | 5,00 | 750 | 15,00 | 25,00 |
| Concentré de protéines de soja[4] | 14,00 | 14,00 | 14,00 | 14,00 | 14,00 |
| Gluten de blé[5] | 9,05 | 9,25 | 9,40 | 9,65 | 10,10 |
| Gluten de maïs[6] | 8,20 | 8,20 | 8,20 | 8,20 | 8,20 |
| Farine de soja 48 | 7,50 | 7,50 | 7,50 | 7,50 | 7,50 |
| Pois entier | 6,15 | 5,75 | 5,40 | 4,75 | 3,70 |
| Huile de poisson | 11,50 | 11,50 | 11,50 | 11,50 | 11,50 |
| Huile de colza | 6,00 | 5,80 | 5,70 | 5,40 | 5,00 |
| Pré-mélange de vitamines et minéraux[7] | 1,50 | 1,50 | 1,50 | 1,50 | 1,50 |
| Lécithine de soja | 1,00 | 1,00 | 1,00 | 1,00 | 1,00 |
| Gomme de guar | 0,20 | 0,20 | 0,20 | 0,20 | 0,20 |
| Antioxydant | 0,20 | 0,20 | 0,20 | 0,20 | 0,20 |
| Propionate de sodium | 0,10 | 0,10 | 0,10 | 0,10 | 0,10 |
| Phosphate Mono Calcique | 1,30 | 1,70 | 2,00 | 2,60 | 3,50 |
| DL-méthionine | 0,30 | 0,30 | 0,30 | 0,40 | 0,50 |
| Oxyde d'yttrium[8] | 0,02 | 0,02 | 0,02 | 0,02 | 0,02 |
| | | | | | |
| Matière sèche (MS), %* | 93,4 ± 0,0 | 93,1 ± 0,0 | 93, ± 0,1 | 95,0 ± 0,0 | 93,2 ± 0,0 |
| Protéine brute, % MS** | 48,5 ± 0,0 | 48,5 ± 0,1 | 48,5 ± 0,0 | 48,5 ± 0,0 | 48,5 ± 0,1 |
| Matières grasses brutes,% MS** | 22,7 ± 0,2 | 22,7 ± 0,1 | 22,6 ± 0,2 | 22,7 ± 0,2 | 22,7 ± 0,2 |
| Ingrédients en %* : | CTRL | Y5 | Y7,5 | Y15 | Y25 |
| Cendre, % MS** | 9,4 ± 0,0 | 8,8 ± 0,0 | 8,7 ± 0,1 | 8,1 ± 0,0 | 7,4 ± 0,0 |
| Chitine, % MS** | 0,06 | 0,46 | 0,66 | 1,26 | 2,06 |

(suite)

| Ingrédients en %* : | CTRL | Y5 | Y7,5 | Y15 | Y25 |
|---|---|---|---|---|---|
| Énergie brute, MJ/kg de MS | 23,2 ± 0,2 | 23,2 ± 0,0 | 23,2 ± 0,0 | 23,2 ± 0,1 | 23,2 ± 0,1 |

* % de matière sèche par rapport au poids total de la composition
** % en poids sec par rapport au poids total de la matière sèche
[1] Farine de poisson péruvienne LT70 : 71 % de protéines brutes (PB), 11% de matières grasses brutes (MGB), EXALMAR, Pérou; [2] Farine de krill : 61% PB, 19% MGB, Aker BioMarine Antarctic AS, Norvège;[3] Super Prime sans viscères : 82% PB, 3.5% MGB, Sopropêche, France; [4]Soycomil P: 62% PB, 0.7% MGB, ADM, Pays-Bas; [5]VITEN: 84.7% PB, 1.3% MGB, ROQUETTE, France; [6] Farine de gluten de maïs : 61% PB, 6% MGB, COPAM, Portugal; [7] PREMIX Lda, Portugal. Vitamines (IU ou mg/kg régime) : Acétate de DL-alpha tocophérol, 100 mg; Bisulfate de sodium et de ménadione, 25 mg; Acétate de rétinyle, 20 000 UI; DL-cholécalciférol, 2000 IU; thiamine, 30 mg; riboflavine, 30 mg; pyridoxine, 20 mg; cyanocobalamine, 0,1 mg; acide nicotinique, 200mg; acide folique, 15 mg; acide ascorbique, 1000 mg; inositol, 500 mg; biotine, 3 mg; pantothénate de calcium, 100 mg; chlorure de choline, 1000 mg, bétaïne, 500mg. Minéraux (g ou mg/kg) : carbonate de cobalt, 0.65mg; sulfate de cuivre, 9 mg; sulfate ferrique, 6 mg; iodure de potassium, 0,5 mg; oxyde de manganèse, 9,6 mg; sélénite de sodium, 0,01 mg; sulfate de zinc, 7,5 mg; chlorure de sodium, 400 mg; carbonate de calcium, 1,86 g; blé excipient ; [8] l'oxyde d'yttrium a été incorporé dans seulement une fraction des aliments utilisés pour les mesures de digestibilité.

Les niveaux de farine de calmar et de krill ont été maintenus constants parmi tous les régimes afin de garantir une haute palatabilité. Des ajustements mineurs sur la formulation des régimes testés ont été faits pour maintenir les conditions isoazotées (protéine brute, 48,5% de MS), isolipidique (22,7% MS) et isoénergétique (énergie brute, 23,2 MJ/ kg MS). Les niveaux de supplémentation en méthionine et en phosphate monocalcique dans les régimes testés ont été ajustés pour correspondre à ceux trouvés dans l'alimentation CTRL.

Les régimes ont été fabriqués par extrusion (tailles des granulés : 1,2 et 2.0mm) par une extrudeuse à deux vis CLEXTRAL BC45 à échelle pilote avec un diamètre de vis de 55,5 mm et une gamme de température de 119 à 123°C. Lors de l'extrusion, tous les lots d'aliments extrudés ont été séchés dans un séchoir à lit fluidisé vibrant (modèle DR100, TGC Extrusion, France). Après refroidissement des granulés, les huiles ont été ajoutées par revêtement sous vide (modèle PG-10VCLAB, Dinnisen, Pays-Bas). Pendant toute la durée de l'essai, les aliments expérimentés ont été stockés à température ambiante, mais dans un emplacement frais et aéré. Des échantillons représentatifs de chaque régime ont été prélevés pour analyse (Tableaux 4-5).

Tableau 4 : Profil en acides aminés des régimes expérimentaux.

| Acides aminés | CTRL | Y5 | Y7,5 | Y15 | Y25 |
|---|---|---|---|---|---|
| Arginine | 4,62 ± 0,23 | 4,53 ± 0,02 | 4,49 ± 0,23 | 4,27 ± 0,09 | 3,89 ± 0,09 |
| Histidine | 1,47 ± 0,11 | 1,56 ± 0,02 | 1,54 ± 0,09 | 1,46 ± 0,07 | 1,50 ± 0,08 |
| Isoleucine | 2,31 ± 0,01 | 2,52 ± 0,01 | 2,53 ± 0,01 | 2,46 ± 0,02 | 2,49 ± 0,00 |
| Leucine | 4,51 ± 0,08 | 4,44 ± 0,01 | 4,68 ± 0,05 | 4,46 ± 0,02 | 4,56 ± 0,01 |
| Lysine | 3,09 ± 0,19 | 3,09 ± 0,01 | 3,02 ± 0,17 | 2,94 ± 0,01 | 2,97 ± 0,03 |
| Thréonine | 2,32 ± 0,03 | 2,37 ± 0,00 | 2,31 ± 0,03 | 2,14 ± 0,05 | 2,15 ± 0,02 |
| Valine | 2,75 ± 0,00 | 2,87 ± 0,02 | 3,00 ± 0,03 | 3,08 ± 0,01 | 3,18 ± 0,01 |
| Méthionine | 1,71 ± 0,15 | 1,71 ± 0,01 | 1,75 ± 0,06 | 1,74 ± 0,02 | 1,63 ± 0,02 |
| Cystéine | 0,35 ± 0,02 | 0,34 ± 0,00 | 0,31 ± 0,02 | 0,33 ± 0,00 | 0,34 ± 0,00 |
| Phénylalanine | 3,30 ± 0,00 | 3,06 ± 0,01 | 2,92 ± 0,15 | 2,85 ± 0,01 | 2,56 ± 0,00 |
| Tyrosine | 2,44 ± 0,11 | 2,48 ± 0,00 | 2,67 ± 0,14 | 2,92 ± 0,04 | 3,14 ± 0,12 |
| Taurine | 0,20 ± 0,01 | 0,20 ± 0,00 | 0,21 ± 0,01 | 0,06 ± 0,00 | 0,04 ± 0,00 |
| Les teneurs sont indiquées en pourcentages en poids sur le poids total de granulés avant séchage. | | | | | |

Tableau 5 : Synthèse du profil en acides gras des régimes expérimentaux.

| Acides gras | CTRL | Y5 | Y7,5 | Y15 | Y25 |
|---|---|---|---|---|---|
| C14:0 | 0,40 ± 0,00 | 0,40 ± 0,00 | 0,38 ± 0,00 | 0,43 ± 0,00 | 0,38 ± 0,00 |
| C16:0 | 1,86 ± 0,01 | 1,89 ± 0,01 | 1,82 ± 0,02 | 2,11 ± 0,01 | 1,94 ± 0,02 |
| C16:1n-7 | 0,48 ± 0,00 | 0,48 ± 0,00 | 0,44 ± 0,00 | 0,50 ± 0,00 | 0,42 ± 0,01 |
| C18:0 | 0,49 ± 0,00 | 0,50 ± 0,01 | 0,47 ± 0,01 | 0,54 ± 0,00 | 0,50 ± 0,01 |
| C18:1n-9 | 1,62 ± 0,01 | 1,74 ± 0,01 | 1,69 ± 0,01 | 2,08 ± 0,01 | 2,06 ± 0,02 |
| C18:1n-7 | 0,26 ± 0,00 | 0,25 ± 0,00 | 0,23 ± 0,00 | 0,25 ± 0,00 | 0,21 ± 0,00 |
| C18:2n-6 | 0,79 ± 0,00 | 0,94 ± 0,01 | 1,05 ± 0,01 | 1,36 ± 0,01 | 1,53 ± 0,02 |
| C18:3n-3 | 0,13 ± 0,00 | 0,13 ± 0,00 | 0,13 ± 0,00 | 0,14 ± 0,00 | 0,12 ± 0,00 |
| C18:4n-3 | 0,10 ± 0,00 | 0,10 ± 0,00 | 0,09 ± 0,00 | 0,10 ± 0,00 | 0,08 ± 0,00 |
| C20:1 n-9 | 0,20 ± 0,00 | 0,19 ± 0,00 | 0,17 ± 0,00 | 0,18 ± 0,00 | 0,14 ± 0,00 |
| C20:4n-6 | 0,14 ± 0,00 | 0,13 ± 0,00 | 0,12 ± 0,00 | 0,14 ± 0,00 | 0,12 ± 0,00 |
| C20:5n-3 | 0,72 ± 0,00 | 0,71 ± 0,01 | 0,65 ± 0,00 | 0,70 ± 0,00 | 0,57 ± 0,01 |
| C22:1 n-11 | 0,14 ± 0,00 | 0,13 ± 0,00 | 0,11 ± 0,00 | 0,12 ± 0,00 | 0,08 ± 0,00 |
| C22:5n-3 | 0,14 ± 0,00 | 0,13 ± 0,00 | 0,12 ± 0,00 | 0,13 ± 0,00 | 0,10 ± 0,00 |
| C22:6n-3 | 1,45 ± 0,01 | 1,44 ± 0,01 | 1,33 ± 0,01 | 1,46 ± 0,01 | 1,21 ± 0,02 |
| Les teneurs sont indiquées en pourcentage en poids sur le poids total de granulés avant séchage. | | | | | |

## 1.3. Essai sur la performance de croissance

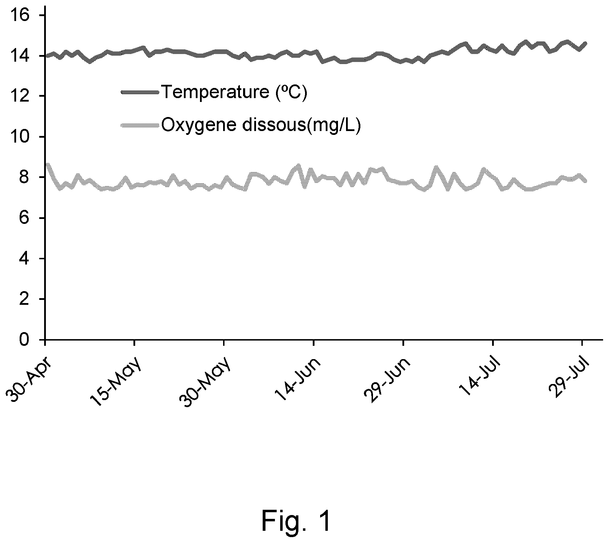

Des groupes en trois exemplaires de 35 truites arc-en-ciel *(Oncorhynchus mykiss),* avec un poids corporel initial (PCI) de 5,01± 0,1 g ont été nourris avec l'un des cinq régimes expérimentaux pendant 90 jours. Les poissons ont grandis dans des réservoirs circulaires en fibre de verre (volume : 250 L) alimentés en eau douce à écoulement continu, à des températures comprises entre 14,1 ± 0,3°C et des niveaux d'oxygène dissous au-dessus de 7,4 mg/L (voir Figure 1). Les poissons ont été soumis par des conditions d'été à des changements de photopériode naturelle (mai-juillet). Les poissons ont été nourris à satiété apparente, à la main, trois fois par jour (9h00, 14h00 et 18h00) en semaine et deux fois par jour les week-ends (10h00 et 16h00), avec le plus grand soin pour éviter le gaspillage d'aliments. L'aliment distribué a été quantifié tout au long de l'étude. Des poissons anesthésiés ont été pesés individuellement au début et à la fin de l'étude et le groupe a été pesé au jour 28 et au jour 60. Au début, 15 poissons du même stock initial ont été échantillonnés et stockés à -20°C pour une analyse ultérieure de la composition corporelle intégrale. Après 90 jours d'alimentation expérimentale, 6 poissons de chaque réservoir ont été échantillonnés dans le même but.

## 1.4. Mesures de digestibilité apparente

À la fin de l'essai de croissance et suivant tous les échantillonnages associés, 12 poissons (poids corporel : 45 g) de chaque réservoir réplique ont été utilisés pour déterminer la digestibilité apparente de la matière sèche, des protéines, des lipides, de l'énergie et du phosphore, par la méthode indirecte avec les régimes identiques contenant l'oxyde d'yttrium (200 mg/kg) comme traceur inerte. Les poissons ont été stockés dans des réservoirs cylindro-coniques (volume : 60 L; taux d'écoulement de l'eau : 3,7 L/min; niveaux d'oxygène dissous supérieures à 6,4 mg/L), à une température de l'eau constante à 14°C. Les poissons ont été adaptés pendant 10 jours aux conditions d'élevage et aux régimes expérimentaux. Ensuite, les poissons ont été nourris une fois par jour (10h00), à la main en léger excès. Après un nettoyage en profondeur des bassins d'élevage pour retirer tous les résidus d'aliments, les matières fécales ont été prélevées quotidiennement pendant les 8 jours suivants en utilisant le système de filtration continu de l'eau de sortie (système Choubert-INRA). Après une collecte quotidienne, les matières fécales ont été congelées à -20°C. Les matières fécales mélangées provenant de chaque groupe de poissons ont été lyophilisées avant l'analyse. Chaque régime alimentaire a été testé en triple exemplaire.

Les coefficients de digestibilité apparente (CDA) des nutriments et de l'énergie alimentaire dans les régimes expérimentaux ont été calculés selon la formule :

$$CDA(\%) = 100 - \left[ \frac{\%\ \text{concentration}\ Y_2O_3\ \text{aliment}}{\%\ \text{concentration}\ Y_2O_3\ \text{feces}} \times \frac{\%\ \text{Energie ou nutriments dans feces}}{\%\ \text{Energie ou nutriments dans aliment}} \right]$$

**1.5. Méthodes analytiques**

Les ingrédients de test, les régimes alimentaires et les matières fécales lyophilisées ont été broyés avant analyse. Les échantillons du corps entier ont été hachés, mélangés, et un échantillon représentatif a été lyophilisé et homogénéisé avec un moulin de laboratoire avant analyse. L'analyse de la composition chimique de l'ingrédient, des régimes alimentaires, des matières fécales et du poisson entier a été faite en utilisant les procédures suivantes : matière sèche après séchage à 105°C pendant 24 h; cendres par combustion à 550°C pendant 12 h; protéine brute (N x 6,25) par une technique de combustion éclair suivie d'une séparation par chromatographie en phase gazeuse et détection de conductivité thermique (LECO FP428) ; la matière grasse par extraction au dichlorométhane (Soxhlet) ; le phosphore total selon la méthode ISO / DIS 6491 en utilisant le réactif vanado-molybdique ; l'énergie brute dans une bombe calorimétrique adiabatique. L'oxyde d'yttrium dans les aliments et les fèces a été déterminé par la méthode ICP-AES.

Pour les analyses d'acides aminés totaux, les ingrédients de test et les régimes de test ont été hydrolysés (6 M de HCL à 116°C pendant 22 h dans des flacons en verre rincés à l'azote), puis dérivatisés avec un réactif fluor AccQ (6-aminoquinolyl-N-hydroxysuccinimidyle) selon la méthode de Tag AccQ (Waters, USA). Les analyses ont été effectuées par une chromatographie en phase liquide à haute performance (HPLC) dans un système d'analyse des acides aminés en phase inverse, en utilisant la norvaline comme étalon interne. Le tryptophane n'a pas été déterminé car il est partiellement détruit par l'hydrolyse acide. Les pics résultants ont été analysés avec le logiciel EMPOWER (Waters, USA). Pour l'analyse des acides gras, les lipides ont été extraits selon la méthode de Folch et al. (1957) et par la suite, la composition en acides gras des filets a été déterminée par une analyse des esters méthyliques par chromatographie en phase gazeuse, selon le mode opératoire de Lepage et Roy (1986).

**1.6. Critère pour l'évaluation de la croissance et l'utilisation des nutriments**

PCI (g): Poids corporel initial.
PCF (g): Poids corporel final.
Taux de croissance spécifique, TCS (% / jour) : (Ln PCF - Ln PCI) x 100/days.
Indice de consommation, IC : ration alimentaire brute / prise de poids.
Apport alimentaire volontaire, AAV (%PC/jour) : (ration alimentaire brute / (PCI+PCF) / 2 / jours) x 100.
Coefficient d'efficacité protéique CEP : prise de poids mouillé / apport en protéine brute.
Rétention (% de l'apport) : 100 x (PCF x teneur finale en nutriments de la carcasse - PCI x teneur initiale en nutriments de la carcasse) / apport en nutriment.

**1.7. Analyse statistique**

Les données sont présentées par la moyenne de trois répétitions ± l'écart type. Les données ont été soumises à une analyse de la variance à un facteur. Avant ANOVA, les valeurs exprimées en % ont été soumises à une transformation de la racine carrée arc sinus. La signification statistique a été testée à un niveau d'une probabilité de 0,05. Tous les tests statistiques ont été effectués en utilisant le logiciel IBM SPSS V21.

**2. Résultats**

**2.1. Performance de croissance**

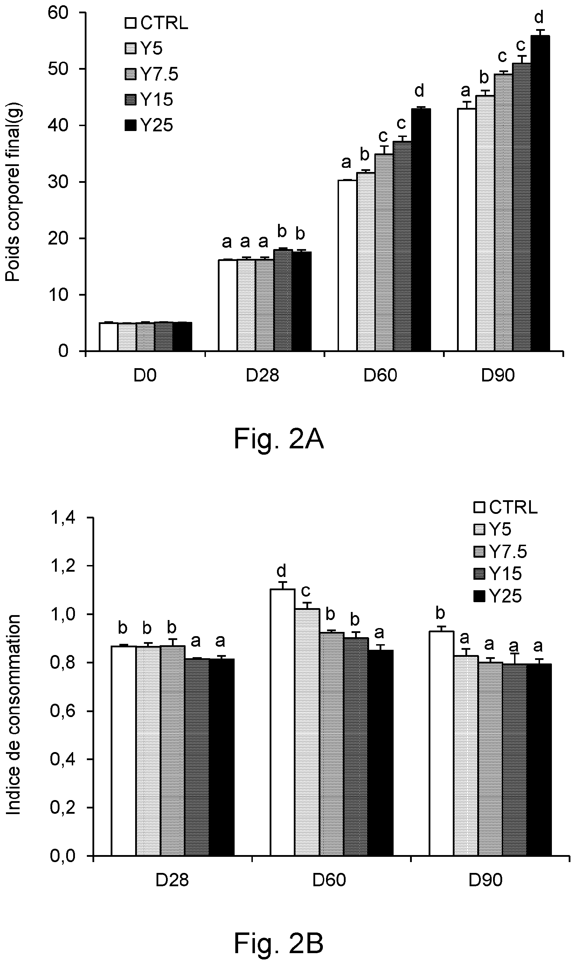

Les données sur les performances de croissance, la conversion alimentaire et l'efficacité protéique de la truite arc-en-ciel nourrie durant 28, 60 et 90 jours avec les régimes expérimentaux sont reportées dans les tableaux 6-8 et la Figure 2. Aucune mortalité n'est survenue au cours de l'essai.

Tableau 6 : Performances de croissance au jour 28.

| Régime | CTRL | Y5 | Y7.5 | Y15 | Y25 |
|---|---|---|---|---|---|
| PCI (g) | 5,0 ± 0,1 | 4,9 ± 0,1 | 5,0 ± 0,1 | 5,1 ± 0,1 | 5,1 ± 0,1 |

(suite)

| Régime | CTRL | Y5 | Y7.5 | Y15 | Y25 |
|---|---|---|---|---|---|
| PCF (g) | 16,1 ± 0,1 [a] | 16,2 ± 0,5 [a] | 16,2 ± 0,5 [a] | 17,9 ± 0,3 [b] | 17,6 ± 0,4 [b] |
| TCS, %/j | 4,19 ± 0,12 [a] | 4,26 ± 0,13 [a] | 4,20 ± 0,07 [a] | 4,50 ± 0,07 [b] | 4,45 ± 0,06 [b] |
| IC | 0,87 ± 0,01 [b] | 0,87 ± 0,02 [b] | 0,87 ± 0,03 [b] | 0,81 ± 0,00 [a] | 0,81 ± 0,01 [a] |
| Apport alimentaire, %PCM/j | 3,27 ± 0,07 | 3,31 ± 0,09 | 3,28 ± 0,09 | 3,25 ± 0,03 | 3,22 ± 0,07 |
| CEP | 2,55 ± 0,02 [a] | 2,56 ± 0,05 [a] | 2,55 ± 0,08 [a] | 2,66 ± 0,01 [ab] | 2,72 ± 0,05 [b] |
| Les valeurs sont les moyennes ± l'écart type (n=3), Les valeurs au sein d'une rangée avec des exposants différents diffèrent de façon significative (P <0,05). | | | | | |

Après 28 jours d'alimentation expérimentale (Tableau 5), les poissons ont plus que triplé leur poids corporel initial. L'apport en aliment était élevé (3,22 - 3,31% PCM/jour) et n'a pas été affecté (P> 0,05) par les doses d'incorporation croissantes en composition selon l'invention. Cette observation suggère que la composition selon l'invention n'a eu aucun effet négatif sur la palatabilité et même qu'elle pourrait compenser l'élimination totale de la farine de poisson sans compromettre l'apport alimentaire. Le taux de croissance a varié de 4,19 à 4,50% / jour. En comparaison avec le traitement CTRL, tandis que les régimes Y5 et Y7,5 n'ont pas affecté le PCF et le TCS, les régimes Y15 et Y25 ont entraîné une augmentation significative (P <0,05) du PCF et du TCS. Les valeurs de l'indice de consommation varient entre 0,81 et 0,87. En comparaison avec le CTRL, l'inclusion de composition selon l'invention à 5 et 7,5% (régimes Y5 et Y7,5%) n'a pas affecté l'IC. Cependant, les niveaux d'inclusion élevés de composition selon l'invention (régimes de Y15 et Y25) ont conduit à une réduction significative de l'IC (P <0,05). Le coefficient d'efficacité protéique (CEP) a varié entre 2,55 et 2,72. Les poissons nourris avec un régime Y25 ont montré une augmentation significative de CEP, par rapport à ceux nourris avec les régimes CTRL, Y5 et Y7,5.

Tableau 7 : Performances de croissance au jour 60.

| Régime | CTRL | Y5 | Y7,5 | Y15 | Y25 |
|---|---|---|---|---|---|
| PCI (g) | 5,0 ± 0,1 | 4,9 ± 0,1 | 5,0 ± 0,1 | 5,1 ± 0,1 | 5,1 ± 0,1 |
| PCF (g) | 30,3 ± 0,1 [a] | 31,6 ± 0,5 [a] | 34,9 ± 1,5 [b] | 37,2 ± 0,9 [c] | 42,9 ± 0,4 [d] |
| TCS, %/j | 3,00 ± 0,04 [a] | 3,10 ± 0,04 [b] | 3,24 ± 0,04 [c] | 3,31 ± 0,05 [c] | 3,57 ± 0,04 [d] |
| IC | 1,10 ± 0,03 [d] | 1,02 ± 0,03 [c] | 0,92 ± 0,01 [b] | 0,90 ± 0,02 [b] | 0,85 ± 0,02 [a] |
| CEP | 2,01 ± 0,06 [a] | 2,17 ± 0,06 [b] | 2,40 ± 0,02 [c] | 2,46 ± 0,06 [cd] | 2,56 ± 0,07 [d] |
| Les valeurs sont les moyennes ± l'écart type (n=3). Les valeurs au sein d'une rangée avec des exposants différents diffèrent de façon significative (P <0,05). | | | | | |

Après 60 jours d'alimentation expérimentale (Tableau 6), les poissons du meilleur traitement performant ont montré une augmentation de 8 fois le poids corporel initial. Le taux de croissance a varié de 3,00 à 3,57 % / jour. En comparaison avec le traitement CTRL, tous les régimes avec la composition selon l'invention ont montré une augmentation significative (P <0,05) du TCS. Les valeurs de l'IC ont varié entre 0,85 et 1,10 et en comparaison avec le CTRL, l'inclusion de la composition selon l'invention à toutes les doses testées a entraîné une réduction significative de l'IC (P <0,05). Le coefficient d'efficacité protéique (CEP) a varié entre 2,01 et 2,56. La valeur la plus basse du CEP a été trouvée chez les poissons nourries avec un régime CTRL, tandis qu'une amélioration du CEP a été étroitement associée aux doses croissantes de la composition selon l'invention.

Tableau 8 : Performances de croissance au jour 90.

| Régime | CTRL | Y5 | Y7,5 | Y15 | Y25 |
|---|---|---|---|---|---|
| PCI (g) | 5,0 ± 0,1 | 4,9 ± 0,1 | 5,0 ± 0,1 | 5,1 ± 0,1 | 5,1 ± 0,1 |
| PCF (g) | 42,9 ± 1,3 [a] | 45,2 ± 1,0 [b] | 49,0 ± 0,6 [c] | 51,0 ± 1,4 [c] | 55,9 ± 1,0 [d] |
| TCS, %/j | 2,39 ± 0,06 [a] | 2,47 ± 0,02 [b] | 2,54 ± 0,03 [b] | 2,56 ± 0,05 [b] | 2,67 ± 0,04 [c] |
| IC | 0,93 ± 0,02 [b] | 0,83 ± 0,03 [a] | 0,80 ± 0,02 [a] | 0,79 ± 0,04 [a] | 0,79 ± 0,02 [a] |

(suite)

| Régime | CTRL | Y5 | Y7,5 | Y15 | Y25 |
|---|---|---|---|---|---|
| CEP | 2,38 ± 0,06 [a] | 2,68 ± 0,10 [b] | 2,76 ± 0,06 [b] | 2,80 ± 0,15 [b] | 2,74 ± 0,08 [b] |
| Les valeurs sont les moyennes ± l'écart type (n=3). Les valeurs au sein d'une rangée avec des exposants différents diffèrent de façon significative (P <0,05). | | | | | |

À la fin de l'essai, 90 jours d'alimentation expérimentale (Tableau 7), les poissons du meilleur traitement performant ont montré une augmentation de 11 fois le poids corporel initial. En comparaison avec le poisson CTRL, ceux nourris les régimes riches en insectes ont montré une augmentation significative du poids corporel final (P <0,05). Cette augmentation a été liée à la dose, avec une augmentation modérée pour le régime Y5, intermédiaire pour Y7,5 et Y15, et la plus élevée pour Y25. Le taux de croissance spécifique (TCS) a varié entre 2,39 et 2,67 % / jour, avec une valeur minimale trouvée chez les poissons nourris avec un régime CTRL, tandis que ceux nourris avec des aliments contenant de la composition selon l'invention ont montré des valeurs de TCS significativement plus élevées (p <0,05). Indépendamment du niveau d'incorporation, la composition selon l'invention a conduit à une réduction significative de l'IC (P <0,05). En comparaison avec le traitement CTRL, tous les régimes de repas d'insectes ont conduit à une augmentation significative des valeurs du CEP (P <0,05).

## 2.2. Composition du corps entier

Les données sur la composition du corps entier de la truite à la fin de l'essai sont présentées dans le Tableau 9. Les traitements alimentaires n'ont eu aucun effet (P> 0,05) sur la teneur en humidité, en protéines, en lipides, en cendres, en phosphore et en énergie du poisson entier.

Tableau 9 : Composition du corps entier de la truite nourrie avec les divers traitements alimentaires.

| Composition corporelle | CTRL | Y5 | Y7,5 | Y15 | Y25 |
|---|---|---|---|---|---|
| Humidité, % | 70,1 ± 0,6 | 70,7 ± 0,4 | 71,1 ± 0,4 | 70,5 ± 0,5 | 70,7 ± 1,2 |
| Protéine, % | 14,8 ± 0,6 | 14,8 ± 0,3 | 15,0 ± 0,5 | 15,2 ± 0,3 | 15,2 ± 0,7 |
| Matière grasse, % | 12,2 ± 0,2 | 11,5 ± 0,4 | 11,0 ± 0,3 | 11,6 ± 0,1 | 11,8 ± 0,9 |
| Cendre, % | 1,9 ± 0,0 | 2,2 ± 0,2 | 2,1 ± 0,3 | 2,1 ± 0,0 | 2,2 ± 0,1 |
| Phosphore, % | 0,4 ± 0,0 | 0,4 ± 0,0 | 0,4 ± 0,0 | 0,4 ± 0,0 | 0,4 ± 0,0 |
| Énergie, kJ/g | 8,2 ± 0,1 | 8,0 ± 0,0 | 8,0 ± 0,0 | 8,0 ± 0,2 | 8,2 ± 0,4 |
| Les pourcentages sont des pourcentages en poids sur le poids total du poisson. Les valeurs sont les moyennes ± l'écart type (n=3). Poisson initial : humidité 75,0%; protéine 14,1%; matières grasses 8,7% ; cendres 2,2%; phosphore 0,4%, énergie 6,7 kJ / g. | | | | | |

2.3. Rétention des nutriments

Les valeurs des nutriments et de la rétention de l'énergie (exprimées en pourcentage de l'apport) sont présentées dans le Tableau 10. En comparaison avec le traitement CTRL, les poissons nourris avec des régimes riches en composition selon l'invention ont montré une augmentation significative de protéine et de la rétention d'énergie (P <0,05). De même, les régimes Y7,5, Y15 et Y25 ont montré une rétention en P significativement plus élevé que le CTRL (P <0,05). La rétention de matière grasse n'a pas été affectée par les régimes alimentaires (P> 0,05).

Tableau 10 : Rétention des nutriments et de l'énergie dans la truite alimentée par les divers régimes alimentaires.

| Rétention, % apport | CTRL | Y5 | Y7,5 | Y15 | Y25 |
|---|---|---|---|---|---|
| Protéine | 35,5 ± 2,5 [a] | 39,8 ± 0,7 [b] | 41,6 ± 0,4 [b] | 42,8 ± 2,2 [b] | 41,9 ± 2,2 [b] |
| Matière grasse | 64,4 ± 2,1 | 68,0 ± 4,9 | 66,8 ± 3,3 | 71,5 ± 3,4 | 70,9 ± 6,7 |
| Phosphore | 30,5 ± 0,7 [a] | 32,7 ± 1,8 [ab] | 34,0 ± 0,7 [b] | 33,9 ± 1,7 [b] | 33,8 ± 1,1 [b] |

(suite)

| Rétention, % apport | CTRL | Y5 | Y7,5 | Y15 | Y25 |
|---|---|---|---|---|---|
| Énergie | 42,0 ± 0,8 [a] | 45,4 ± 1,6 [b] | 47,1 ± 1,4 [b] | 47,8 ± 1,8 [b] | 48,0 ± 2,9 [b] |
| Les valeurs sont les moyennes ± l'écart type (n=3). Les valeurs au sein d'une rangée avec des exposants différents diffèrent de façon significative (P <0,05). | | | | | |

### 2.4. Digestibilité apparente

La composition des matières fécales collectées à partir de la truite nourrie par les divers traitements alimentaires est présentée au Tableau 11.

Tableau 11 : Composition des matières fécales de la truite nourrie avec les divers régimes alimentaires.

| Composition de la matière fécale | CTRL | Y5 | Y7,5 | Y15 | Y25 |
|---|---|---|---|---|---|
| Oxyde d'yttrium, (mg/kg) | 1384 ± 39 | 1395 ± 94 | 1415 ± 61 | 1369 ± 62 | 1411 ± 43 |
| Protéine, % MS* | 19,63 ± 0,06 | 19,67 ± 0,24 | 19,76 ± 0,34 | 19,70 ± 0,38 | 19,20 ± 0,41 |
| Matières grasses, % MS* | 4,37 ± 0,06 | 4,33 ± 0,19 | 4,28 ± 0,24 | 4,30 ± 0,06 | 4,20 ± 0,33 |
| Phosphore, % MS* | 2,64 ± 0,06 | 2,77 ± 0,08 | 2,65 ± 0,10 | 2,54 ± 0,15 | 2,62 ± 0,09 |
| Énergie, kJ/g MS | 23,24 ± 0,16 | 23,14 ± 0,40 | 23,47 ± 0,47 | 22,88 ± 0,16 | 23,09 ± 0,16 |
| *Pourcentage en poids sur le poids total de matière fécale sèche. Les valeurs sont les moyennes ± l'écart type (n=3). | | | | | |

Les coefficients de digestibilité apparente (CDA %) pour les différents nutriments et l'énergie sont présentés dans le Tableau 12. L'augmentation des doses d'incorporation de la composition selon l'invention n'a eu aucun effet significatif (P> 0,05) sur la digestibilité apparente de la matière sèche, des protéines, de la matière grasse, du phosphore et de l'énergie.

Tableau 12 : Digestibilité apparente des nutriments et de l'énergie dans la truite.

| CDA % | CTRL | Y5 | Y7,5 | Y15 | Y25 |
|---|---|---|---|---|---|
| Matière sèche | 84,2 ± 0,4 | 84,2 ± 1,0 | 84,3 ± 0,7 | 84,0 ± 0,7 | 84,3 ± 0,5 |
| Protéine | 93,6 ± 0,2 | 93,6 ± 0,4 | 93,6 ± 0,2 | 93,5 ± 0,4 | 93,8 ± 0,1 |
| Matière grasse | 97,0 ± 0,1 | 97,0 ± 0,1 | 97,0 ± 0,2 | 97,0 ± 0,2 | 97,1 ± 0,3 |
| Phosphore, % d'apport | 69,9 ± 1,4 | 68,3 ± 1,5 | 70,5 ± 2,4 | 71,4 ± 2,9 | 70,3 ± 1,8 |
| Énergie, % d'apport | 84,1 ± 0,4 | 84,3 ± 0,8 | 84,1 ± 1,0 | 84,2 ± 0,6 | 84,4 ± 0,6 |
| Les valeurs sont les moyennes ± l'écart type (n=3). | | | | | |

### 3. Conclusion

À la fin des 90 jours d'alimentation expérimentale, la performance globale de la croissance peut être considérée comme très satisfaisante et dans une gamme plus élevée pour les jeunes truites arc-en-ciel, avec des valeurs de TCS pour la durée totale du test variant entre 2,4 et 2,7% / jour. Dans les traitements les plus performants, les poissons ont montré une augmentation de 11 fois leur poids corporel initial. Les taux de conversion alimentaire parmi les traitements ont varié entre 0,79 et 0,93, ce qui suggère une bonne adéquation nutritionnelle des aliments et des bonnes pratiques alimentaires.

Les données expérimentales générées dans cet exemple permettent d'affirmer que :

- L'incorporation de doses croissantes de composition selon l'invention (5, 7,5, 15 et 25%) avec une réduction concomitante de la farine de poisson a été progressivement liée à une augmentation significative du poids corporel du poisson.

- Tous les régimes contenant la composition selon l'invention ont montré une amélioration significative des TCS, IC et du CEP.
- Les doses d'incorporation croissantes de composition selon l'invention n'ont eu aucun effet sur la composition du corps entier de la truite.
- Les doses d'incorporation croissantes de composition selon l'invention n'ont eu aucun effet sur la digestibilité apparente de la matière sèche, des protéines, des lipides, de phosphore et de l'énergie dans les différents régimes expérimentaux.
- Les protéines, le phosphore et la rétention d'énergie ont été renforcés chez les truites nourries avec des aliments comportant de la composition selon l'invention.

En général, la composition selon l'invention mise en œuvre dans cet exemple pourrait remplacer efficacement 100% de la farine de poisson dans le régime alimentaire des truites arc-en-ciel juvéniles avec des effets positifs sur l'IC et la performance globale de la croissance.

**EXEMPLE** 5 : **Procédés** avec **ou** sans broyage **préalable** au pressage

Procédé avec pressage uniquement

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis passées dans une presse de type bi-vis. Un gâteau de presse est ainsi obtenu.

Procédé avec un broyage suivi d'un pressage

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. Un gâteau de presse est ainsi obtenu.

Mesure du taux de lipides

On place 2 g d'échantillon dans un bécher, on y ajoute 0,2 g de $Na_2SO_4$ et 15 mL de $CHCl_3$/MeOH (2/1 v/v). L'ensemble est placé sous agitation magnétique pendant 20 minutes, ensuite la solution est filtrée, le résidu est placé de nouveau dans le bécher avec 10 mL de $CHCl_3$/MeOH (2/1 v/v). L'ensemble est placé sous agitation magnétique pendant 15 minutes, ensuite la solution est filtrée, les phases solvants sont réunies et évaporées à poids constant. La teneur en lipides est déterminée comme pourcentage de masse après extraction-évaporation par rapport à la masse initiale de l'échantillon (2 g).

Conclusion :

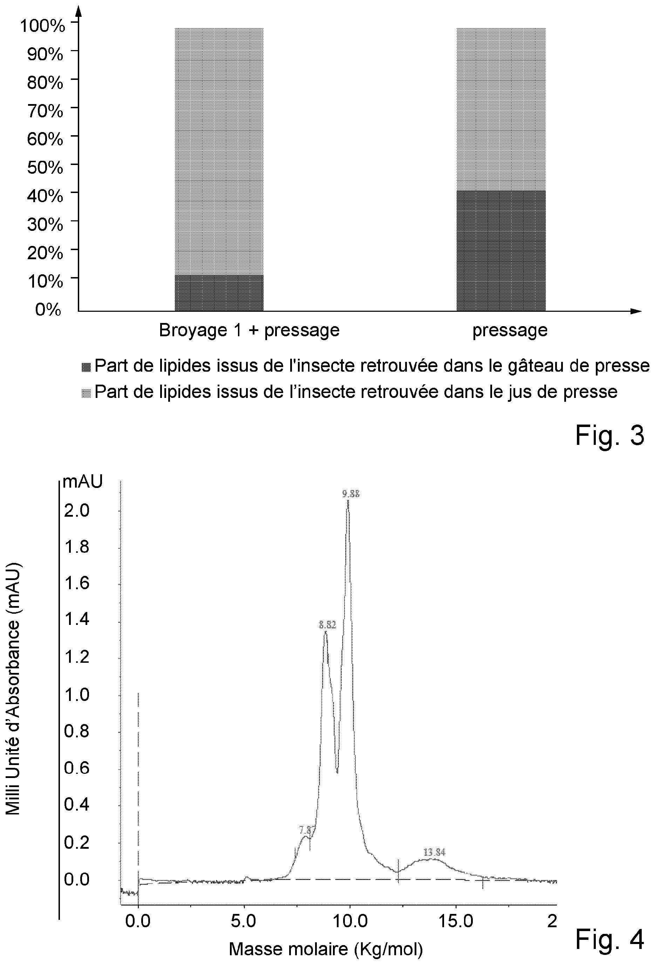

L'importance du broyage en amont du pressage a été étudiée (Figure 3). Il apparait ainsi clairement que la répartition des lipides entre le gâteau et le jus de presse est bien plus efficace, 12,9 *versus* 87,1 contre 42,7 *versus* 57,3, lorsqu'un broyage préalable a été réalisé.

**EXEMPLE 6** : **Analyse de la taille des protéines solubles de la composition selon l'invention.**

Un échantillon de 100 mg de la composition préparée à l'Exemple 1 a été placé dans 10 mL de tampon phosphate NaCl (pH 7,4, 0,137 mM). L'échantillon a été agité durant 1 minute (vortex), puis centrifugé à 900 g durant 1 min. Suite à la centrifugation, l'échantillon a été filtré sur une membrane de 0,45 µm. L'analyse de la taille des protéines solubles a été réalisée à l'aide d'un système de chromatographie par exclusion stérique, avec une colonne Nucleogel GFC-300. Un tampon phosphate NaCl (pH 7,4, 0,137 mM) a été utilisé comme éluant. Le débit était de 1,0 mL/min. La détection a été réalisée par un détecteur UV à 280 nm.

Les résultats de l'analyse sont présentés dans la Figure 4 et résumés dans le Tableau 13 ci-dessous.

Tableau 13 : Répartition des tailles des protéines solubles contenues dans la composition préparée à l'Exemple 1

| **Taille des protéines** (kg/mol) | **Abondance relative (%)** |
|---|---|
| 6,5 à 12,4 | 74 ,4 |

(suite)

| Taille des protéines (kg/mol) | Abondance relative (%) |
|---|---|
| 12,4 à 29 | 20,5 |
| 29 à 66 | 5,1 |

Les résultats montrent qu'environ 74,4 % des protéines solubles présentes dans la composition selon l'invention ont une masse molaire inférieure à 12400 g/mol (ou Da, Daltons)

**Revendications**

1. Composition comportant au moins 67% en poids de protéines brutes, au moins 5% en poids de chitine, les pourcentages en poids étant donnés sur le poids total de composition, et 85% en poids de protéines digestibles sur le poids total de protéines brutes.

2. Composition selon la revendication 1, comportant des cendres à une teneur inférieure ou égale à 4% en poids sur le poids total de composition.

3. Composition selon la revendication 1 ou 2, comportant de la matière grasse à une teneur comprise entre 5 et 20% en poids sur le poids total de composition.

4. Composition selon l'une quelconque des revendications 1 à 3, obtenue à partir d'insectes.

5. Composition selon l'une quelconques des revendications 1 à 4, dont le taux d'humidité résiduel est compris entre 2 et 15%.

6. Composition selon l'une quelconque des revendications 1 à 5, comportant entre 30 et 60% en poids de protéines solubles par rapport au poids total de protéines brutes, dans laquelle au moins 50% des protéines solubles ont une taille inférieure ou égale à 12400g/mol.

7. Procédé de préparation d'une composition selon l'une quelconque des revendications 4 à 6, comportant les étapes suivantes :

    i) abattage d'insectes,
    ii) pressage des insectes pour obtenir un gâteau de presse,
    iii) séchage du gâteau de presse, et
    iv) broyage du gâteau de presse.

8. Procédé selon la revendication 7, comportant les étapes suivantes,

    i) abattage des insectes,
    ii) pressage des insectes pour obtenir un gâteau de presse,
    iii) séchage du gâteau de presse, et
    iv) broyage du gâteau de presse,

    dans lequel l'étape de pressage est précédée d'une étape de broyage des insectes.

9. Procédé selon la revendication 7, comportant les étapes suivantes,

    i) abattage des insectes,
    ii) pressage des insectes pour obtenir un gâteau de presse,
    iii) séchage du gâteau de presse, et
    iv) broyage du gâteau de presse,

    dans lequel l'étape de pressage est réalisée à chaud.

**10.** Procédé selon la revendication 7, comportant les étapes suivantes,

> i) abattage des insectes,
> ii) pressage des insectes pour obtenir un gâteau de presse,
> iii) séchage du gâteau de presse, et
> iv) broyage du gâteau de presse,

dans lequel l'étape de broyage du gâteau de presse est réalisée à une taille de particules comprise entre 300 µm et 1 mm.

**11.** Utilisation de la composition selon l'une quelconque des revendications 1 à 6, dans l'alimentation humaine ou animale.

**12.** Utilisation selon la revendication 11, dans laquelle la composition est utilisée en remplacement d'une farine protéique.

**Patentansprüche**

**1.** Zusammensetzung, welche umfasst: mindestens 67 Gewichtsprozent Rohproteine, mindestens 5 Gewichtsprozent Chitin, wobei die Gewichtsprozente bezogen auf das Gesamtgewicht der Zusammensetzung angegeben sind, und 85 Gewichtsprozent verdauliche Proteine, bezogen auf das Gesamtgewicht der Rohproteine.

**2.** Zusammensetzung nach Anspruch 1, welche Asche zu einem Anteil von weniger als oder gleich 4 Gewichtsprozent, bezogen auf das Gesamtgewicht der Zusammensetzung, umfasst.

**3.** Zusammensetzung nach Anspruch 1 oder 2, welche Fett zu einem Anteil umfasst, der zwischen 5 und 20 Gewichtsprozent, bezogen auf das Gesamtgewicht der Zusammensetzung, liegt.

**4.** Zusammensetzung nach einem der Ansprüche 1 bis 3, erhalten ausgehend von Insekten.

**5.** Zusammensetzung nach einem der Ansprüche 1 bis 4, deren Restfeuchtigkeitsgehalt zwischen 2 und 15 Prozent liegt.

**6.** Zusammensetzung nach einem der Ansprüche 1 bis 5, welche im Verhältnis zum Gesamtgewicht an Rohproteinen zwischen 30 und 60 Gewichtsprozent lösliche Proteine umfasst, und in welcher mindestens 50 Prozent der löslichen Proteine eine Größe von weniger als oder gleich 12400 g/mol aufweisen.

**7.** Verfahren zur Herstellung einer Zusammensetzung nach einem der Ansprüche 4 bis 6, welches die folgenden Schritte umfasst:

> i) Töten von Insekten,
> ii) Pressen der Insekten, um einen Presskuchen zu erhalten,
> iii) Trocknen des Presskuchens, und
> iv) Zerkleinern des Presskuchens.

**8.** Verfahren nach Anspruch 7, welches die folgenden Schritte umfasst:

> i) Töten von Insekten,
> ii) Pressen der Insekten, um einen Presskuchen zu erhalten,
> iii) Trocknen des Presskuchens, und
> iv) Zerkleinern des Presskuchens,

und in welchem dem Pressschritt ein Schritt der Zerkleinerung der Insekten vorangeht.

**9.** Verfahren nach Anspruch 7, welches die folgenden Schritte umfasst:

> i) Töten von Insekten,
> ii) Pressen der Insekten, um einen Presskuchen zu erhalten,

iii) Trocknen des Presskuchens, und
iv) Zerkleinern des Presskuchens,

und in welchem der Pressschritt in der Wärme durchgeführt wird.

10. Verfahren nach Anspruch 7, welches die folgenden Schritte umfasst:

i) Töten von Insekten,
ii) Pressen der Insekten, um einen Presskuchen zu erhalten,
iii) Trocknen des Presskuchens, und
iv) Zerkleinern des Presskuchens,

und in welchem der Schritt der Zerkleinerung des Presskuchens bis zu einer Partikelgröße durchgeführt wird, die zwischen 300 $\mu$m und 1 mm liegt.

11. Verwendung der Zusammensetzung nach einem der Ansprüche 1 bis 6 in der menschlichen oder tierischen Ernährung.

12. Verwendung nach Anspruch 11, bei der die Zusammensetzung als Ersatz für ein Proteinmehl verwendet wird.


**Claims**

1. Composition comprising at least 67% by weight of crude proteins, at least 5% by weight of chitin, the percentages by weight being given with respect to the total weight of composition, and 85% by weight of digestible proteins with respect to the total weight of crude proteins.

2. Composition according to claim 1, comprising an ash content less than or equal to 4% by weight with respect to the total weight of composition.

3. Composition according to claim 1 or 2, comprising a fat content comprised between 5 and 20% by weight with respect to the total weight of composition.

4. Composition according to any one of claims 1 to 3, obtained from insects.

5. Composition according to any one of claims 1 to 4, of which the residual moisture content is comprised between 2 and 15%.

6. Composition according to any one of claims 1 to 5, comprising between 30 and 60% by weight of soluble proteins with respect to the total weight of crude proteins, in which at least 50% of the soluble proteins have a size less than or equal to 12400 g/mol.

7. Process for preparing a composition according to any one of claims 4 to 6, comprising the following steps:

i) killing insects,
ii) pressing the insects in order to obtain a press cake,
iii) drying the press cake, and
iv) grinding the press cake.

8. Process according to claim 7, comprising the following steps,

i) killing the insects,
ii) pressing the insects in order to obtain a press cake,
iii) drying the press cake, and
iv) grinding the press cake,

in which the pressing step is preceded by a step of grinding the insects.

**9.** Process according to claim 7, comprising the following steps,

  i) killing the insects,
  ii) pressing the insects in order to obtain a press cake,
  iii) drying the press cake, and
  iv) grinding the press cake,

in which the pressing step is carried out under heat.

**10.** Process according to claim 7, comprising the following steps,

  i) killing the insects,
  ii) pressing the insects in order to obtain a press cake,
  iii) drying the press cake, and
  iv) grinding the press cake,

in which the step of grinding the press cake is carried out to a particle size comprised between 300 $\mu$m and 1 mm.

**11.** Use of the composition according to any one of claims 1 to 6, in human or animal nutrition.

**12.** Use according to claim 11, in which the composition is used instead of a protein-containing flour.

Fig. 1

Fig. 2A

Fig. 2B

Part de lipides issus de l'insecte retrouvée dans le gâteau de presse
Part de lipides issus de l'insecte retrouvée dans le jus de presse

Fig. 3

Fig. 4

**RÉFÉRENCES CITÉES DANS LA DESCRIPTION**

**Documents brevets cités dans la description**

- US 20080075818 A

**Littérature non-brevet citée dans la description**

- **SANDRA G.F. BUKKENS.** The nutritional value of edible insects. *Ecology of Food and Nutrition,* 1997, vol. 36 (2-4), 287-319